# EUROPEAN PATENT APPLICATION

(11) **EP 3 249 460 A2**
(43) Date of publication of application: **29.11.2017**
(21) Application number: 17169101.7
(22) Date of filing: 02.05.2017
(51) Int. Cl.: G03B 3/02, G03B 15/14, A61K 49/00, G03B 15/03, G03B 17/02, H04N 5/232

(54) **METHODS AND DEVICES FOR DETECTING BIOFILMS ON THE EYELID MARGIN**

(30) Priority: 02.05.2016 US 201662330657 P
(71) Applicant: Rynerson, James, M., Alvaton, KY 42122 (US)
(72) Inventor: Rynerson, James, M., Alvaton, KY 42122 (US)
(74) Representative: Findlay, Alice Rosemary

(57) **Abstract**

A composition for detecting biofilm on an eyelid margin includes a dye that binds to the biofilm and a solution that is biocompatible with the eye. A method for detecting biofilm on an eyelid margin of an eye includes applying a solution to the eye or the eyelid margin so that a dye in the solution binds to the biofilm. A handheld camera (10) for detecting biofilm on an eyelid margin of an eye includes a housing (12), an image sensor (46), and a lens (44) including a focal length determined by a distance between the lens (44) and the eyelid margin. A method for detecting biofilm on an eyelid margin includes capturing an image of the eyelid margin using a handheld camera (10) and determining if the biofilm is present on the eyelid margin. A system for detecting biofilm on an eyelid margin of an eye includes a composition and a handheld camera (10).

## Description

The present invention relates generally to a method and instrument for detecting an ocular disorder and, more particularly, to an instrument and a composition for detecting biofilms on the eyelid margin and methods of using said instrument and composition.

Ocular disorders such as those relating to eyelid margin disease are particularly common pathological conditions of the ocular adenexa. By way of example, these disorders include blepharitis, meibomitis, and dry eye syndrome. Eyelid margin disease usually includes a buildup of debris on the eyelid margin of the eye of an individual. The debris may include bacteria, scurf, mucus, oils, and other secreted fluids. Despite advances in ophthalmology and medical treatments in general, the recommended treatments for these exemplary common ocular disorders has remained essentially unchanged for decades.

Eyelid margin disease may be the most common eye disease in the world and, unfortunately, one of the most poorly understood and under-treated eye diseases of our time. There is no single diagnostic test for eyelid margin disease, and therefore the exact prevalence of this disease is difficult to determine. Most prevalence studies have failed to recognize blepharitis and dry eye disease as differing stages of the same disease process, and thus there is a significant amount of overlap when evaluating the prevalence of this disease. Based on the self-reporting of dry eyes in the Beaver Dam Offspring cohort, the prevalence of dry eye was 14.5 percent (18.9 percent in women and 10.5 percent in men). Other studies have estimated dry eye prevalence at 5 to 32 percent of the population age 50 years and over. Self-reporting is a poor protocol for estimating dry eye disease, because early meibomian gland disease is often asymptomatic. Studies on blepharitis prevalence have focused on doctor reporting, thereby evaluating their existing patient populations to determine prevalence. This method is obviously skewed towards patients already under the care of an eye doctor, so is pre-selected towards patients with existing symptoms. These numbers range from 40% to 65%. The complexity of over-lapping symptoms makes determination difficult as well. When factoring the number of contact lens wearers in the US, currently around 40 million, it is easy to see that eyelid margin disease is a significant eye problem worldwide, with a need for better understanding and treatment of this chronic disease.

It is well accepted that dry eye and blepharitis are inflammatory-based diseases. Indeed, a prominent eye drop medication, Restasis® is based on suppressing existing inflammation. A better approach would be to identify a source of inflammation that can account for the many presentations of DEBS. In doing so, the nature of this disease may be better understood and better treatment options may be formulated to address the source of inflammation. There exists a need for a medication that addresses the source of inflammation thereby preventing inflammation from occurring in the first place.

Historically, blepharitis has been treated by advising patients to perform home lid hygiene regimes, which are ineffective and difficult for most patients to do. There is a multitude of home lid hygiene products such as surfactant-based lid wipes, hypochlorous acid and warm compresses. Dry eye disease treatments have been mostly ameliorative, with a multitude of various artificial tears on the market, and more recently, in-office treatments such as Lipiflow®, Meiboflow®, manual meibomian gland expression, and BlephEx®, which is an in-office treatment that allows doctors to completely remove lid margin biofilms. None of these treatments address a recurrence of the source of inflammation, and must be repeated at regular intervals.

There is a need for a method and apparatus for use in diagnosing ocular disorders, such eyelid margin diseases, that addresses present challenges and characteristics such as those discussed above.

The present invention provides improved compositions, devices, and methods for detecting biofilm on an eyelid margin of an eye. An aspect of the invention is directed to a composition for detecting biofilm on an eyelid margin. The composition includes a dye that binds to the biofilm and a solution that is biocompatible with the eye.

Another aspect of the invention is directed to a method for detecting biofilm on an eyelid margin of an eye. The method includes applying a solution to the eye or the eyelid margin so that a dye in the solution binds to the biofilm.

Another aspect of the invention is directed to a handheld camera for detecting biofilm on an eyelid margin of an eye. The handheld camera includes a housing, an image sensor, and a lens including a focal length determined by a distance between the lens and the eyelid margin.

Another aspect of the invention is directed to a method for detecting biofilm on an eyelid margin. The method includes capturing an image of the eyelid margin using a handheld camera and determining if the biofilm is present on the eyelid margin.

Another aspect of the invention is directed to a system for detecting biofilm on an eyelid margin of an eye. The system includes a composition and a handheld camera. The composition comprises a dye that binds to the biofilm, and a solution that is biocompatible with the eye. The handheld camera comprises a housing, an image sensor, and a lens including a focal length determined by a distance between the lens and the eyelid margin.

The invention will now be further described by way of example with reference to the accompanying drawings, in which:
FIG. 1 is a perspective view of an embodiment of a device in accordance with an aspect of the invention.
FIG. 2 is a perspective view of the device of FIG. 1 with the cup and a portion of the housing removed for clarity.
FIG. 3 is a perspective view of the device of FIG. 1 shown in cross section.
FIG. 4A is a cross-sectional view of the device of FIG. 1.
FIG. 4B is an enlarged cross-sectional view, similar to FIG. 4A, showing the lens in a different position.
FIG. 5 is an exploded view of the device of FIG. 1.
FIG. 6 is an exploded view of a portion of the device of FIG. 1.

The Detailed Description refers to accompanying drawings to illustrate exemplary embodiments consistent with the present disclosure. References in the Detailed Description to "one exemplary embodiment," "an exemplary embodiment," an "example exemplary embodiment," etc., indicate that the exemplary embodiment described may include a particular feature, structure, or characteristic, but every exemplary embodiment may not necessarily include the particular feature, structure, or characteristic. Moreover, such phrases are not necessarily referring to the same exemplary embodiment. Further, when a particular feature, structure, or characteristic is described in connection with an exemplary embodiment, and it is within the knowledge of those skilled in the art(s) to affect such feature, structure, or characteristic in connection with other exemplary embodiments whether or not explicitly described, such other embodiments, so affected, are intended to be suggested and included in this description.

The exemplary embodiments described herein are provided for illustrative purposes, and are not limiting. Other exemplary embodiments are possible, and modifications may be made to the exemplary embodiments within the spirit and scope of the present disclosure. Therefore, the Detailed Description is not meant to limit the present disclosure.

The Detailed Description of the exemplary embodiments will so fully reveal the general nature of the present disclosure that others can, by applying knowledge of those skilled in the relevant art(s), readily modify and/or adapt for various applications such exemplary embodiments, without undue experimentation, without departing from the spirit and scope of the present disclosure. Therefore, such adaptations and modifications are intended to be within the meaning and plurality of equivalents of the exemplary embodiments based upon the teaching and guidance presented herein. It is to be understood that the phraseology or terminology herein is for the purpose of description and not of limitation, such that the terminology or phraseology of the present specification is to be interpreted by those skilled in relevant art(s) in light of the teachings herein.

An aspect of the invention is directed to a method of detecting biofilms on the eyelid margin by using a dye that specifically binds to biofilms. A composition for detecting biofilms may be in the form of drops that are suitable for use in and around the eye. Compositions for detecting biofilms may include dyes that are similar to dyes used in mouth rinses that show plaque on teeth. Suitable dyes include phloxine B, D & C Red 28, and FD & C Blue. One of ordinary skill in the art will appreciate that exact components of the composition may vary based on the intended use.

In use, the composition stains, or otherwise increases the visibility of, any biofilm on the subject's eyelids. The dye may be administered in formulations that include additional excipients and solvents as are known in the art for use in compounds being applied to the eye or eyelid margin. For example, the dye compositions may include one or more of the following: balanced salt solutions, water, gelling solutions, and stabilizers. In an embodiment, the dye is in an isotonic solution to reduce any irritation of the eye by the application of the composition.

In embodiments of the invention, dye compositions are administered to the eye lid margin to detect biofilms on the eyelid margin. The dye composition may be administered topically. In an embodiment, the dye composition is administered as an eye drop. Additionally or alternatively, the dye composition may be applied directly to the eyelid margin for more specific application. In an embodiment, the dye composition is administered as a gel or cream directly to the eyelid margin, such as with a fingertip or swab.

Compositions for detecting biofilms according to embodiments of the invention have a variety of applications. In one application, a doctor may use a composition to help with the education process of the patient. Another exemplary use includes a doctor using a composition to determine the efficacy of a biofilm removal procedure such as a BlephEx® microblepharoexfoliation treatment. Additionally, a patient can use a composition at home to determine if a biofilm is present and whether a removal treatment should be scheduled.

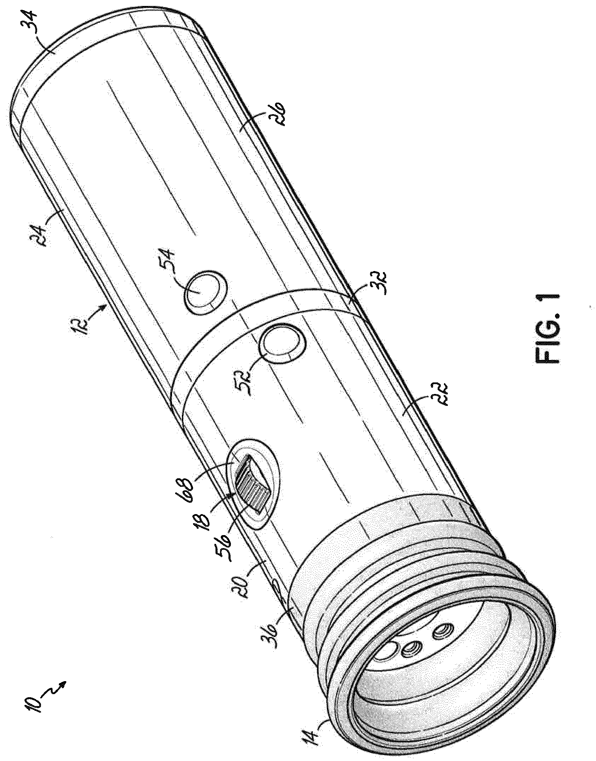

An aspect of the invention is directed to device for detecting biofilms on the eyelid margin. With reference to FIGS. 1-6, in an embodiment, a camera 10 is specifically designed to capture images of the eyelid margin of a patient. The camera 10 includes an elongate outer housing 12 and a cup 14 for fitting over an eye. The camera 10 further includes an imaging system 16 and a focusing mechanism 18. The camera 10 is used to capture an image allowing the user to see debris, which may include scurf, dead cells, and biofilm, that is adherent to the upper and/or lower eyelid margin. The camera 10 is described in more detail below.

Still referring to FIGS. 1-5, in an embodiment, the outer housing 12 is segmented and includes two distal halves 20, 22 and two proximal halves 24, 26. The distal halves 20, 22 each include a proximal lip 28 into which corresponding distal lips 30 of the proximal halves 24, 26 extends. A cover 32 is positioned over the lips 28 to secure each of the distal and proximal halves 20, 22, 24, 26 together. The outer housing 12 further includes an end cap 34, which couples to the proximal halves 24, 26.

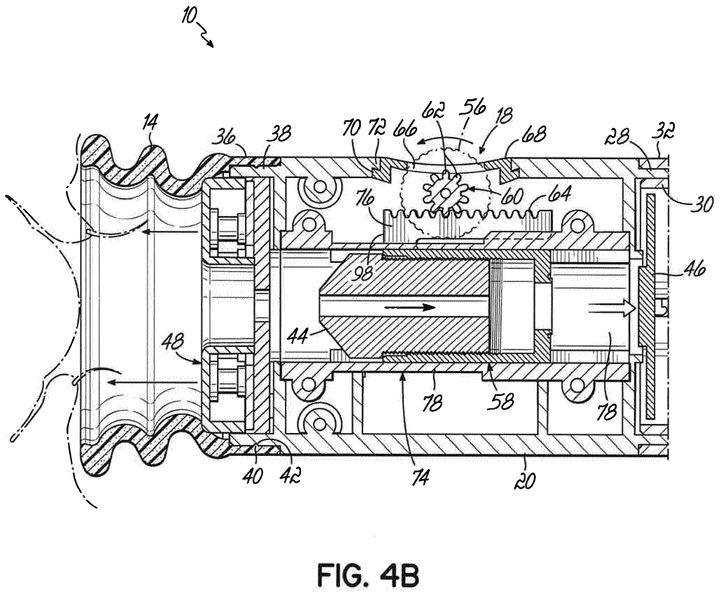

With reference to FIGS. 1 and 4A-6, the cup 14 is coupled to the outer housing 12. In an embodiment, the cup 14 includes a collar 36 that fits around a distal lip 38 of the distal halves 20, 22 of the outer housing 12. The collar 36 may include a projection 40 that engages a corresponding notch 42 on the distal lip 38 when the cup 14 is positioned on the outer housing 12. During use, the cup 14 is placed over the subject's eye (shown in phantom in FIG. 4B, not necessarily to scale). The cup 14 is designed so that pressing the camera 10 against the subject's face does not cause pain. Additionally, the cup 14 substantially prevents light from outside of the cup 14 from interfering with the image taken by the camera 10. An exemplary material for the cup 14 is rubber, such as silicone. It should be recognized that other suitable materials may be used.

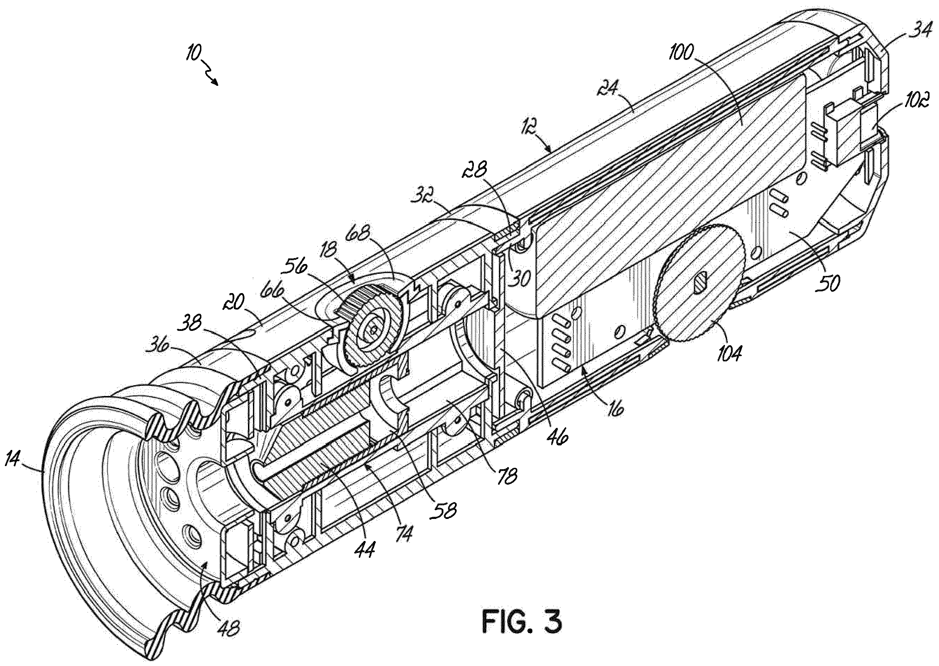

With reference to FIGS. 3-6, in an embodiment, the imaging system 16 includes a lens 44, a light source 48, an image sensor 46, and an imaging circuit board 50. The image sensor 46 may be an electronic sensor capable of capturing an image. Exemplary image sensors include charge-coupled device ("CCD") sensors, a complementary metal-oxide-semiconductor ("CMOS") sensor, and a contact image sensor ("CIS"). The imaging system 16 further includes a capture button 52 that, when pressed, triggers the capture of an image. Embodiments of the camera 10 may also include a digital zoom feature. A digital zoom button 54 may be pressed to cycle through a series of magnification levels. The camera 10 may be capable of zooming from a magnification of 5X up to a magnification of 80X, for example. Additionally, an exemplary digital zoom may have a magnification of 3X.

Referring again to FIGS. 1-6, in an embodiment, the focusing mechanism 18 includes an axially aligned focusing thumbwheel 56 and a lens carriage 58, which is coupled to the lens 44. The thumbwheel 56 is rotatable to adjust the focus of the camera 10. More specifically, the thumbwheel 56 includes a projection 60 including teeth 62 that cooperate with a corresponding series of teeth 64 on the lens carriage 58. The teeth 62, 64 are movable relative to each other in a rack and pinion configuration. Axial rotation of the thumbwheel 56 translates into linear motion of the lens carriage 58. In this configuration, movement of the thumbwheel 56 towards the cup 14 causes the lens carriage 58 to move away from the cup 14 and vice versa. The thumbwheel 56 extends through an opening 66 in the outer housing 12. The opening 66 may be larger in area compared to the portion of the thumbwheel 56 that extends therethrough. A thumbwheel cover 68 is positioned over the thumbwheel 56 and extends across the opening 66. The thumbwheel cover 68 rests in a recess 69 in the outer housing 12. The thumbwheel cover 68 is secured to the outer housing 12 via a projection 70 that is in contact with a ledge 72 of the outer housing 12.

The focal length of the camera 10 is determined by the distance between the lens 44 and the eyelid margin. The focal length may be greater than that of conventional handheld microscopes. For example, the focal length of the lens 44 may be between about 0-30 mm. The axially aligned focusing thumbwheel 56 improves the ability of a user to adjust the focus with the same hand holding the camera 10 against the subject's eye. In that regard, axial movement of the thumbwheel 56 results in an adjustment of the focus of the image. Thus, only one finger is needed to focus the image. In contrast, traditional focusing components are cylindrical and extend around a handheld camera housing. Radial movement of the focusing component, such as a focusing ring, around the handheld camera is required to adjust the focus. Such a traditional focusing component would significantly impact the ease of using the camera 10 with one hand.

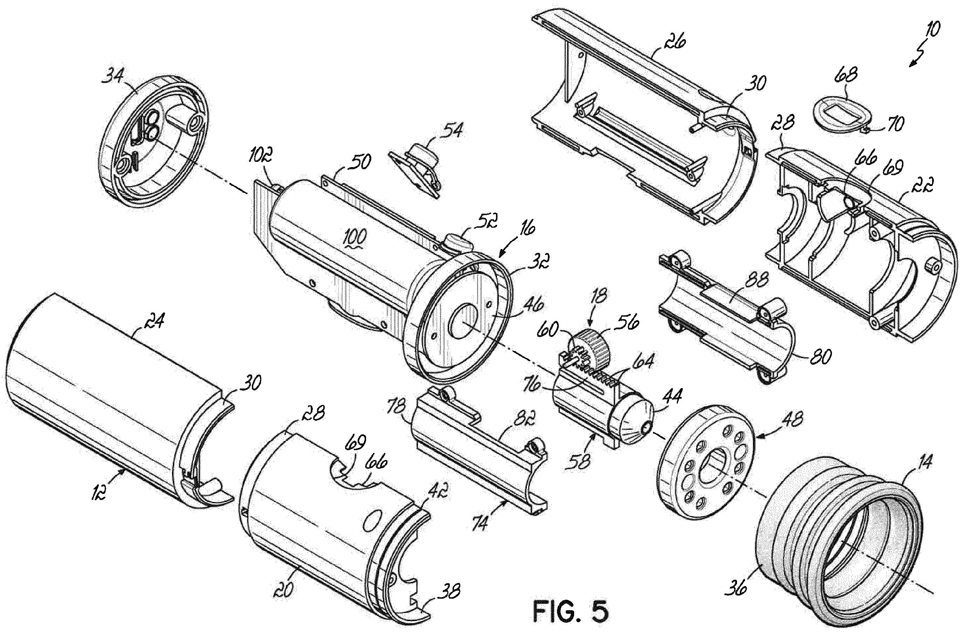

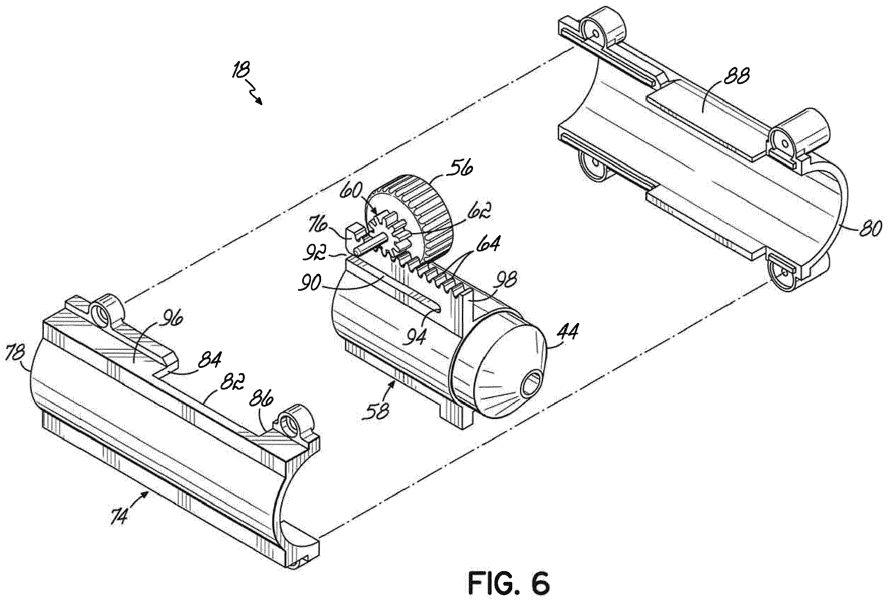

Still referring to FIGS. 3-5, in an embodiment, the lens carriage 58 is partially enclosed by a carriage housing 74. An arm 76 of the lens carriage 58 extends above or outside of the carriage housing 74. The carriage housing 74 is segmented and includes halves 78, 80, which may be held in place by the outer housing 12. As shown best in FIGS. 5 and 6, the carriage housing half 78 includes a recess 82. The recess 82 includes a first edge 84 and a second edge 86 and is configured to receive an arm 76 of the lens carriage 58. The carriage housing half 80 includes a projection 88, which extends to the arm 76 when the lens carriage 58 is enclosed by the carriage housing 74. The arm 76 includes an aperture 90 having an open end 92 and a closed end 94. The aperture 90 is sized to receive a surface 96 that extends proximally from the first edge 84 of the recess 82. When the lens carriage 58 moves away from the cup 14, the open end 92 of the aperture 90 moves proximally over the surface 96. When the closed end 94 comes into contact with the first edge 84 of the recess 82, further proximal motion of the lens carriage 58 is prevented. When a distal end 98 of the arm 76 comes into contact with the second edge 86 of the recess, further distal motion of the lens carriage 58 is prevented.

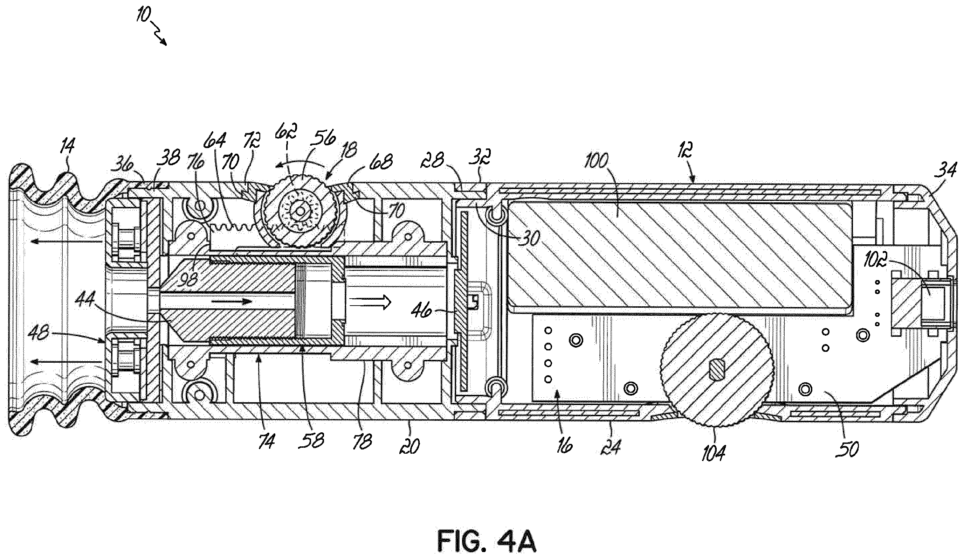

With further reference to FIG. 4A, the camera 10 includes a power source 100 and a charging port 102. The power source 100 may be charged by connecting the camera 10 to an external power source (not shown) using the charging port 102. It should be recognized that the charging port 102 may have a variety of configurations depending on the power source. In an embodiment, the charging port 102 may be a USB port.

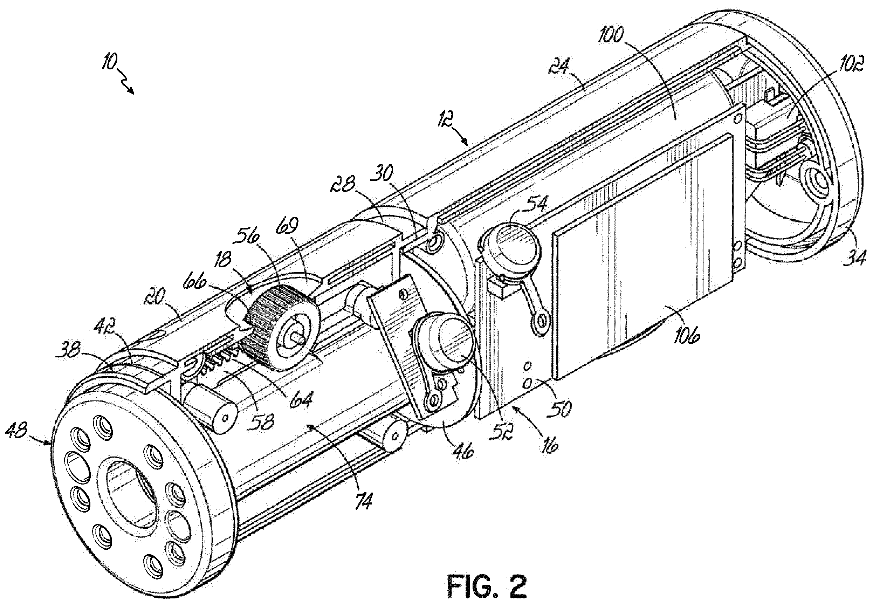

With reference to FIGS. 2-4A, the camera 10 includes an on/off component and a light intensity adjustor. In the illustrated embodiment, a dial 104 serves both purposes. When the dial 104 is rotated to one extreme, the camera 10 is off. When a user rotates the dial 104 away from the extreme, the camera 10 turns on using power from the power source 100. If the user rotates the dial 104 further in the same direction, the light intensity from the light source 48 will increase. Thus, the user may adjust the light intensity to a desired intensity depending on the particular application.

In an aspect of the present invention, Wi-Fi functionality may be incorporated into the camera 10 so that high magnification, high resolution images may be sent to an electronic device such as a smartphone. In an embodiment, the camera 10 includes a Wi-Fi module board 106 as shown in FIG. 2.

During use, the user rotates the dial 104 to turn on the camera 10. Next, the camera 10 is placed such that the cup 14 contacts the face of the patient, encircling the eye. The user may adjust the light intensity using the dial 104 and the zoom of the image using the digital zoom button 54. To focus the image, the user may rotate the thumbwheel 56 causing the lens carriage 58 and, thus, the lens 44 to move towards or away from the patient's eye. When the user is satisfied with the light intensity, zoom, and focus of the image, the image may be captured by pressing the capture button 52. As described above, the image may be wirelessly transferred to an electronic device (not shown) for display and evaluation.

An aspect of the invention is directed to kit for detecting biofilms on the eyelid margin. The biofilm detection dye and a device for detecting biofilms on the eyelid margin (e.g., the camera 10) may be used in concert with one another or separately.

While the present invention has been illustrated by the description of one or more embodiments thereof, and while the embodiments have been described in considerable detail, additional advantages and modifications will readily appear to those skilled in the art.

## Claims

1. A composition for detecting a biofilm on an eyelid margin of an eye comprising:
a dye that binds to the biofilm; and
a solution that is biocompatible with the eye.

2. The composition of claim 1 wherein the solution is an isotonic solution.

3. The composition of either claim 1 or claim 2 wherein the dye includes at least one of phloxine B, D & C Red 28, or FD & C Blue.

4. A handheld camera for detecting biofilm on an eyelid margin of an eye comprising:
a housing;
an image sensor; and
a lens including a focal length determined by a distance between the lens and the eyelid margin.

5. The handheld camera of claim 4 further comprising:
a focusing mechanism for adjusting a focus of the handheld camera.

6. The handheld camera of claim 5 further comprising:
an axially aligned focusing thumbwheel; and
a lens carriage coupled to the lens and movably engaged with the focusing thumbwheel,
wherein axial movement of the focusing thumbwheel moves the lens carriage toward or away from the eyelid margin.

7. The handheld camera of any one of claims 4 to 6 further comprising:
a cup coupled to the housing configured to be positioned on a face of a subject when the handheld camera is in use.

8. The handheld camera of any one of claims 4 to 7 further comprising:
a light source.

9. The handheld camera of claim 8 further comprising:
an adjustor to increase or decrease a light intensity from the light source.

10. The handheld camera of any one of claims 4 to 9 further comprising:
a button for capturing an image.

11. The handheld camera of any one of claims 4 to 10 further comprising:
a button for digitally zooming in or out on an image.

12. A method of detecting a biofilm on an eyelid margin of an eye comprising:
capturing an image of the eyelid margin using a handheld camera;
determining if the biofilm is present on the eyelid margin.

13. The method of claim 12 further comprising:
focusing the handheld camera using an axially aligned focusing thumbwheel.

14. A system for detecting biofilm on an eyelid margin of an eye comprising:
a composition comprising:
a dye that binds to the biofilm; and
a solution that is biocompatible with the eye; and
a handheld camera comprising:
a housing;
an image sensor; and
a lens including a focal length determined by a distance between the lens and the eyelid margin.

15. The system of claim 14, wherein the handheld camera further comprises:
an axially aligned focusing thumbwheel; and
a lens carriage coupled to the lens and movably engaged with the focusing thumbwheel,
wherein axial movement of the focusing thumbwheel moves the lens carriage toward or away from the eyelid margin.
